(19) Europäisches Patentamt
European Patent Office
Office européen des brevets

(11) Veröffentlichungsnummer : **0 004 255**
**B1**

(12) # EUROPÄISCHE PATENTSCHRIFT

(45) Veröffentlichungstag der Patentschrift : **13.01.82**

(21) Anmeldenummer : **79810024.4**

(22) Anmeldetag : **12.03.79**

(51) Int. Cl.³ : **C 07 D221/08**, A 61 K 31/435// C07D265/12

(54) **4-Hydroxy-naphthopyridin-2(1H)-on-3-carbonsäuren und deren Ester, Verfahren zu ihrer Herstellung und diese enthaltende pharmazeutische Zusammensetzungen.**

(30) Priorität : **13.03.78 US 885628**

(43) Veröffentlichungstag der Anmeldung : **19.09.79 (Patentblatt 79/19)**

(45) Bekanntmachung des Hinweises auf die Patenterteilung : **13.01.82 Patentblatt 82/02**

(84) Benannte Vertragsstaaten : **BE CH DE FR GB IT LU NL SE**

(56) Entgegenhaltungen : **Keine**

(73) Patentinhaber : **SANDOZ AG**
**Lichtstrasse 35**
**CH-4002 Basel (CH)**

(72) Erfinder : **Hardtmann, Goetz Eduard**
**12 Lynnfield Drive**
**Morristown N.J. 07960 (US)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 004 255 B1

Imprimerie Jouve, 18, rue St-Denis, 75001 Paris, France

4-Hydroxy-naphthpyridin-2(1H)-on-3-carbonsäuren und deren Ester, Verfahren zu ihrer Herstellung und diese enthaltende pharmazeutische Zusammensetzungen

Die Erfindung betrifft 4-Hydroxy-naphthpyridin-2-(1H)-on-3-carbonsäuren und deren Ester, ihre Herstellung und ihre Verwendung als pharmakologische Wirkstoffe, besonders als anti-allergische Mittel.

Gegenstand der Erfindung sind Verbindungen der allgemeinen Formel I

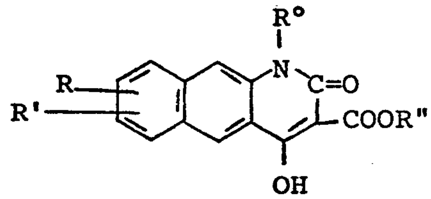

(I)

in der

R° ein Wasserstoffatom, ($C_3$—$C_6$)-Alkyl, ($C_3$—$C_6$)-Alkenyl, ($C_3$—$C_6$)-Alkinyl, ($C_3$—$C_6$)-Cycloalkyl, ($C_3$—$C_6$)-Cycloalkyl-($C_1$—$C_2$)-Alkyl oder

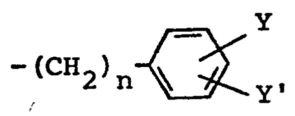

bedeutet,

wobei die ungesättigten Stellen im Alkenyl oder Alkinyl sich nicht auf das α-Kohlenstoffatom beziehen,

R″ ein Wasserstoff atom oder ($C_1$—$C_4$)-Alkyl bedeutet,

n 0 oder 1 ist,

Y und Y′ unabhängig voneinander ein Wasserstoff-, Fluor-, Chlor-, oder Bromatom, ($C_1$—$C_3$)-Alkyl oder ($C_1$—$C_3$)-Alkoxy oder Trifluormethyl bedeuten, und R und R′ jeweils ein Wasserstoff-, Fluor-, Chlor- oder Bromatom, ($C_1$—$C_4$)-Alkyl oder ($C_1$—$C_4$)-Alkoxy bedeuten, und deren Mono- und Disalze, welche pharmazeutisch akzeptable Kationen enthalten.

Erfindungsgemässe Verbindungen, in denen R″ ($C_1$—$C_4$)-Alkyl bedeutet, werden hergestellt indem eine Verbindung der allgemeinen Formel II

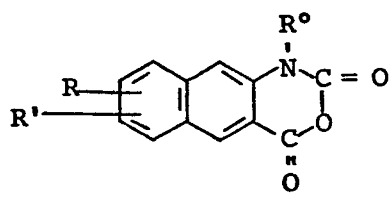

(II)

in der

R, R° und R′ die obenerwähnten Bedeutungen haben, mit einer Verbindung der allgemeinen Formel III

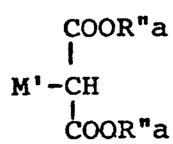

(III)

in der

$R''_a$ ($C_1$—$C_4$)-Alkyl und M′ ein Alkalimetallatom bedeutet, umgesetzt wird.

Die Herstellung der verbindungen I, in denen R″ ($C_1$—$C_4$)-Alkyl ($R''_a$) bedeutet, wird vorzugsweise in einem inerten organischen Lösungsmittel, z.B. Dimethylacetamid und bei einer Temperatur von 0 bis 150 °C, vorzugsweise von 60 bis 130 °C, ausgeführt. Wenn das erhaltene Produkt als 4-Alkalimetallsalz vorliegt, kann daraus durch neutrale oder saure Hydrolyse die freie Säure der allgemeinen Formel I gebildet werden.

Die Verbindungen der allgemeinen Formel III können aus den entsprechenden bekannten Dialkylmalonaten hergestellt werden. Diese werden dazu mit einer Alkalimetallbase, z.B. Natriumhydrid, in einem inerten organischen Lösungsmittel, z.B. Dimethylacetamid, behandelt.

Die erfindungsgemässen Verbindungen, in welchen R″ ($C_1$—$C_4$)-Alkyl bedeutet und deren sauren 4-Hydroxyreste neutralisiert und daher als Monosalz vorhanden sind, werden vorzugsweise aus freien Säuren der allgemeinen Formel I, in der R″ ($C_1$—$C_4$)-Alkyl bedeutet, nach bekannten Methoden hergestellt, z.B. durch Umsetzung mit einer Base, wie verdünnte Natronlauge, in einem mit Wasser mischbaren Lösungsmittel.

Die Verbindungen der allgemeinen Formel I, in der R″ ein Wasserstoff-atom bedeutet und die auch sonst in freier Säureform vorhanden sind, werden aus entsprechenden Verbindungen I, in denen R″ einen sehr labilen Alkylrest, vorzugsweise t-Butyl, darstellt und die als 4-Monosalz oder vorzugsweise als freie Säure vorliegen, durch eine konventionelle Behandlung mit einem Säurekatalysator bei milder Temperatur hergestellt. Die Temperatur wird dabei zwischen − 20 und + 60 °C, vorzugsweise zwischen − 10 und + 35 °C gehalten, damit die Verbindung I nicht decarboxyliert werden. Für die Behandlung mit Säure können die dafür konventionellen Typen verwendet werden, wie Schwefelsäure, Chlorwasserstoffsäure und vorzugsweise Perchlorsäure. Die Spaltung wird zweckmässig in für solchen Umsetzungen konventionellen Lösungsmittelsystemen ausgeführt, z.B. in einem mit Wasser mischbaren organischen Lösungsmittel, das keine Hydroxylgruppen enthält, wie Tetrahydrofuran oder vorzugsweise Acetonitril, wobei das System vorzugsweise einer sehr geringe Menge wasser enthält.

Die Verbindungen der allgemeinen Formel I können in der Disalzform hergestellt werden, indem eine Verbindung I, worin R″ = ($C_1$—$C_4$)-Alkyl bedeutet mit einer Base mit verseifender Wirkung, z.B. Natriumhydroxid, in wässrigem Medium, z.B. Wasser oder wässrigem Aethanol, bei Temperaturen von 40 bis 150 °C, vorzugsweise bei 80 bis 120 °C, behandelt wird. Die Gewinnung kann ebenfalls nach bekannten Methoden erfolgen, wobei Ansäuren vermieden werden soll, damit keine Decarboxylierung stattfindet. Fals die Ausgangsverbindung I in freier Säureform vorliegt, werden mindestens 2 Mol Base, in allgemeinen 2 Mol oder mehr verwendet. Dabei wird auch der Substituent in Stellung 4 beeinflusst und ein Basekation in dieser Stellung eingeführt, falls die Ausgangsverbindung als freie Säure vorliegt, oder werden andere Verbindungen I in die Disalzform übergeführt, wobei die beiden salzbildenden Kationen die gleichen sind oder, wenn die Ausgangsverbindung als 4-Monosalz vorliegt, verschieden sein können, wobei bekannte Faktoren, wie die Base und deren Menge, einen wichtigen Einfluss ausüben. Viele und verschiedene Basen können dabei verwendet werden, wie Alkalimetallhydroxide, Erdalkalimetallhydroxide und Ammonium- und Tetraalkylammonium-hydroxide. Die Base ist jedoch vorzugsweise ein Alkalimetallhydroxid und die Ausgangsverbindung vorzugsweise eine freie Säure, damit die beiden Kationen im Di-salz vom gleichen Alkalimetall, vorzugsweise Natrium oder Kalium, stammen.

Die Verbindungen der allgemeinen Formel I können in ihrer freien Säureform ebenfalls mit 1 Mol jeder gewünschten Base, vorzugsweise ein Alkalimetallhydroxid, wie Natriumhydroxid oder Kaliumhydroxid, behandelt werden. Das geschieht in einem geeigneten Lösungsmittel, wie Wasser oder wässrigem Aethanol, bei einer Temperatur die mindestens bis etwa 150 °C reicht, jedoch vorzugsweise zwischen 0 und 60 °C liegt. Dabei werden die erfindungsgemässen Verbindungen als Monosalz erhalten in dem, durch Tautomerie zwischen den nebeneinander liegenden sauren Gruppen 4-Hydroxy und 3-Carboxyl, das salzbildende Kation ionisch mit beiden Gruppen verbunden und in bekannter Weise damit in einen sechsgliedrigren Ring aufgenommen ist.

Die Verbindung der allgemeinen Formel II, in der R°, R und R′ jeweils ein Wasserstoffatom bedeuten, ist bekannt. Die anderen Verbindungen in denen R° ein Wasserstoffatom bedeutet, können nach bekannten Methoden aus bekannten Ausgangsmaterialien hergestellt werden. Verbindungen der allgemeinen Formel II, in denen R° eine andere Bedeutung als ein Wasserstoffatom hat, sind nicht bekannt und stellen wertvolle neue Zwischenprodukte dar. Solche Verbindungen II können hergestellt werden aus entsprechenden Verbindungen, in denen R° ein Wasserstoffatom oder einen Metallrest bedeutet, z.B. aus einer Verbindung der allgemeinen Formel IIA

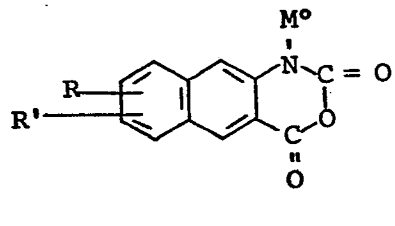

(IIA)

in der

R und R′ die obenerwähnten Bedeutungen haben und M° ein Wasserstoffatom oder ein monovalentes Metallatom, wie Natrium oder Kalium, bedeutet, durch Umsetzung mit einer Verbindung der allgemeinen Formel IV

$$XR^{\circ}_{n} \quad \text{(IV)}$$

worin $R^{\circ}_{n}$ die gleiche Bedeutung wie R°, mit Ausnahme eines Wasserstoffatoms, hat und X ein Chlor-, Brom- oder Jodatom, vorzugsweise ein Bromatom bedeutet. Die Herstellung der Verbindungen II, worin

$R^{o}$ eine andere Bedeutung als ein Wasserstoffatom hat, aus Verbindungen IIA und IV kann nach bekannten Methoden bei Temperaturen von 0 bis 100 °C, vorzugsweise von 20 bis 50 °C ausgeführt werden. Die Umsetzung wird angemessen in einem inerten organischen Lösungsmittel von bekanntem Typ, z.B. Dimethylacetamid, durchgeführt. Vorzugsweise wird die Umsetzung mit einer Verbindung IIA, worin $M^{o}$ ein Alkalimetallatom darstellt, ausgeführt. Eine solche Verbindung wird in bekannter Weise hergestellt, indem eine Verbindung, worin $M^{o}$ ein Wasserstoffatom bedeutet, mit einer starken Base, wie Alkalimetallhydrid, z.B. Natriumhybrid, umgesetzt wird. Falls die Verbindung IIA worin $M^{o}$ ein Wasserstoffatom bedeutet, verwendet wird, wird die Umsetzung in Anwesenheit einer Starken Base, z.B. Alkalimetallalkoxid oder -hydroxid, ausgeführt.

Die Verbindungen der allgemeinen Formel I, entweder als Ester, freie Säure oder als Mono- oder Disalz, zeigen eine Di-natriumchromoglycat (DSCG)-ähnliche Wirkung, besonders eine Wirkung, die die Freisetzung des Histamins hemmt. Aufgrund dieser Wirkung können sie als Antiallergika, beispielsweise zur Behandlung von allergischem Asthma, angewendet werden. Die DSCG-ähnliche Wirkung kann mit dem passiven cutanen Anaphylaxietest an der Ratte nachgewiesen werden. Weibliche Ratten (180-200 g) werden mit 1 mg Eialbumin (Merck Nr. 976) und 200 mg $Al(OH)_3$ in 1 ml einer physiologischen Salzlösung subcutan und mit 0,5 ml Haemophiluspertussis-Impfstoff ($4 \times 10^{10}$ Organismen/ml) intraperitoneal sensibilisiert. 14 Tage später werden die Tiere dekapitiert, das Blut zentrifugiert und das Serum gesammelt und tiefgefroren. An vier Stellen der Rückenhaut von nicht vorbehandelten weiblichen Ratten wird 0,1 ml des 1:200 verdünnten obigen Serums (Antiserum) injiziert. 24 Stunden später wird den Ratten je 0,1 bis 5,6 mg/kg i.v. oder 0,1 bis 100 mg/kg p.o. der Testverbindung verabreicht und entweder direkt oder nach 5 bis 30 min, falls intravenös, oder 15 bis 60 min, falls oral verabreicht, Eialbumin (5 mg/ml) in physiologischer Salzlösung, die 0,25 % Evans Blue als Farbstoff (Merck Nr. 3169) enthält, intravenös eingespritzt. Das Eialbumin löst eine anaphylaktische Hautreaktion aus, deren Intensität dem Ausmass der Farbstoffdiffusion in das die vier sensibilisierten Stellen umringende Gewebe proportional ist. 30 min nach Verabreichen des Eialbumins werden die Ratten mit Aether getötet. Die Rückenhaut der Tiere wird abgelöst und die Durchmesser der blauen Flecken von der Hautunterseite her gemessen. Jede Dosis der Testverbindung wird in vier bis sechs Ratten untersucht und der durchschnittliche Durchmesser mit dem Mittelwert, der bei vier mit Lösungsmittel behandelten Ratten bestimmt wurde, verglichen. Der Prozentsatz der Hemmung wird dem Prozentsatz des durchschnittlichen Durchmessers in den Versuchstieren, bezogen auf der Mittelwert in den Kontrolltieren, gleichgestellt.

Die DSCG-ähnliche Aktivität, besonders die Wirkung, die die Freisetzung des Histamins hemmt, kann an der Hemmung der Histaminfreisetzung in Test der peritonealen Rattemastzellen bestätigt werden, welcher grundsätzlich von Kusner et al. in Pharmacol. Exp. Therap. *184* 41-46 (1973) beschrieben und der mit der folgenden Aenderung durchgeführt wurde : Nach Sedimentieren der Mastzellen durch Zentrifugieren bei 350 × g und 4 °C, werden die Sedimente in 1 ml HBSS (Hanks balanced salt solution) die bei pH 6,9 gepuffert wurde, aufgenommen und gepoolt. Die erhaltene Suspension wird zentrifugiert, erneut mit HBSS gewaschen und sedimentiert. Die so gereinigten Mastzellen werden als 2 ml Suspensionen in HBSS zubereitet. An diese Suspensionen werden jeweils 2 ml HBSS zugefügt, um die spontane Freisetzung des Histamins zu bestimmen, oder 2 ml HBSS und 2,24 Mg der Verbindung 48/80 (N-Methylhomoanisylaminformaldehydkondensat), ein Histaminfreisetzer von Burroughs Wellcome and Co. Ind., Tuckahoe, N.Y. USA, um die 48/80 induzierte Histaminfreisetzung zu bestimmen, oder 2 ml HBSS mit 2,24 Mg 48/80 und 18 bis 180 mg/ml der Testverbindung, zur Bestimmung der 48/80 induzierten Histaminfreisetzung in Anwesenheit der Testverbindung. Die 48/80 induzierte Histaminfreisetzung minus die spontane Histaminfreisetzung wird als 100 %-ige Histaminfreisetzung genommen. Die 48/80 induzierte Histaminefreisetzung in Anwesenheit der Testverbindung minus die spontane Histaminfreisetzung wird sodann mit dem 100 % Wert verglichen, und daraus kann der Prozentsatz der Hemmung durch die Testverbindung berechnet werden. Die Histaminbestimmung wird in bekannter Weise ausgeführt, z.B. wie in der vorher erwähnten Publikation von Kusner oder gemäss Kusner und Herzig in Advances in Automated Analysis, 429 (1971) beschrieben wurde.

Für den obengenannten Gebrauch wird die Dosis durch die zu verwendende Verbindung, die Verabreichungsweise und die Behandlung bestimmt. Sie liegt zwischen 0,3 bis 100 mg/kg des tierischen Körpergewichts und wird zweckmässig in Teilmengen zwei bis vier mal pro Tag oder in verzögerter Abgabeform verabreicht. Für grössere Säugetiere beträgt die tägliche Gesamtdosis etwa 20 bis 400 mg des Wirkstoffs. Sie wird mit einem festen oder flüssigen pharmazeutischen konventionellen Träger vermischt. Teilmengen enthalten 5 bis 200 mg des Wirkstoffs, vermischt mit einem festen oder flüssigen pharmazeutischen Träger. Die erfindungsgemässe Behandlung der allergischen Symptone basiert auf die Hemmung der Histaminfreisetzung und ist daher prinzipiell symptomatisch. Die DSCG-ähnliche Aktivität schliesst ein, dass die Verbindungen zur prophylaktischen Behandlung von allergischen Symptomen eingesetzt können, wobei auch die gute orale Aktivität bezüglich DSCG von Vorteil ist.

Erfindungsgemässe pharmazeutische Zusammensetzungen, brauchbar für die Behandlung von allergischen Symptomen, die aus der Freisetzung von Histamin hervorgehen, enthalten eine Verbindung der allgemeinen Formel I (als freie Säure oder als Salz) als Wirkstoff und einen oder mehrere konventionelle pharmazeutisch akzeptable Träger und gegebenenfalls noch andere konventionelle Zusätze. Solche Zusammensetzungen können konventionelle oral verabreichbare Formen sein, wie Tabletten, Kapseln, Granulate, dispergierbare Puder, Elixiere, Sirupe Suspensionen, oder konventionelle

parenteral verabreichbare Formen, wie injizierbare sterile Lösungen, Suspensionen, z.B. eine sterile injizierbare wässrige Suspension. Solche Zusammensetzungen können nach bekannten pharmazeutischen Herstellungsmethoden zubereitet werden. Die Verbindungen können auch durch Inhalation in dazu geeigneten Zusammensetzungen verabreicht werden. In allgemeinen enthalten Zusammensetzungen zur oralen oder parenteralen Anwendung oder zur Inhalation 1 bis 90 % und meistens 3 bis 70 % des Wirkstoffes in Kombination mit dem Trägerstoff. Feste Dosierungsformen für orale Anwendung, z.B. Tabletten oder Kapseln, werden bevorzugt.

Eine repräsentative Formulierung, die sich zwei bis vier mal pro Tag zur prophylaktischen Behandlung von allergischem Asthma eignet, ist eine Kapsel, die nach bekannten Methoden hergestellt wird und folgende Zusammensetzung aufweist :

| Komponenten | Gewicht (mg) |
|---|---|
| 1-Allyl-4-hydroxy-naphthpyridin-2(1H)-on-3-carbonsaüre-ethylester | 40 |
| Kaolin | 210 |

Die folgenden Beispiele illustrieren die Erfindung :

## Beispiel 1

1-Allyl-4-hydroxy-naphthpyridin-2(1H)-on-3-carbonsäure-ethylester.

a) 1,3-Dioxo-haphth [4,5-b] oxazin :

Eine Suspension von 21 g 3-Amino-2-naphtoesäure in 125 ml Ethylchlorformat wird 24 Stunden unter Rückfluss gekocht und dann filtriert. Der gebildete Niederschlag wird zweimal mit Ethanol und einmal mit Ether gewaschen und liefert die Titelverbindung unter a) mit Smp > 300 °C.

b) 1-Allyl-1,3-dioxo-naphth [4,5-b] oxazin :

An eine Suspension von 13.1 g 1,3-Dioxo-naphth [4,5-b] oxazin in 200 ml. Dimethylacetamid wird portionenweise 3 g 50 %-iges, mit Pentan gewaschenes Natriumhydrid zugefügt, wonach zuerst 30 Min. bei Zimmertemperatur gerührt, dann 8.0 g Allylbromid zugefügt und schliesslich noch 24 Stunden bei Zimmertemperatur gerührt wird. Das Lösungsmittel wird im Vakuum entfernt, wonach Wasser hinzugefügt wird. Der erhaltene ölige Feststoff wird mit Ethylacetat extrahiert, getrocknet, mit Aktivkohle behandelt und im Vakuum konzentriert, wodurch ein öliger Feststoff entsteht, der in Methylendichlorid gelöst wird. Das Methylendichlorid wird durch Ether ersetzt, wonach die Titelverbindung unter b) mit Smp. 170-173 °C erhalten wird.

c) 1-Allyl-4-hydroxy-napthpyridin-2(1H)-on-3-carbonsäure-ethyl-ester :

Zu einer Lösung von 1,9 g Diäthylmalonat in 50 ml. Dimethylacetamid wird portionenweise 600 mg. 50 %-iges, mit Pentan gewaschenes Natriumhydrid zugefügt, wonach bei 25 °C und dann noch kurz bei 120 °C gerührt wird. Zur erhaltenen Lösung wird 3.0 g 1-Allyl-1,3-dioxo-naphth [4,5-b]-oxazin in 40 ml Dimethylacetamid zugefügt, wonach 3 Stunden bei 120 °C gerührt wird. Das Dimethylacetamid wird im Vakuum entfernt und Wasser wird zugefügt. Das Gemisch wird mit Methylendichlorid gewaschen und mit 2N HCl angesäuert. Das Gemisch wird mit Methylendichlorid extrahiert. Die organische Schicht wird getrocknet und im Vakuum eingedampft. Der feste Rückstand wird in Methylendichlorid umkristallisiert (Zugabe von Ether) und liefert die Titelverbindung unter c) mit Smp. 149-152 °C.

## Beispiel 2

Gemäss dem Verfahrensschritt b) im Beispiel 1 werden die folgenden Verbindungen hergestellt :

a) 1-Methyl-1,3-dioxo-naphth [4,5-b] oxazin,
b) 1-(p-Fluorbenzyl)-1,3-dioxo-naphth [4,5-b] oxazin,
c) 1-Phenyl-1,3-dioxo-naphth [4,5-b] oxazin, und
d) 1-Cyclopropylmethyl-1,3-dioxo-napth [4,5-b] oxazin.

## Beispiel 3

Die folgenden Verbindungen werden gemäss Verfahrensschritt c) des Beispiels 1 hergestellt :
a) 1-Methyl-4-hydroxy-naphthpyridin-2(1H)-on-3-carbonsäure-ethylester,
b) 1-(p-Fluorbenzyl)-4-hydroxy-naphpyridin-2-(1H)-on-3-carbonsäure-ethylester,
c) 1-Phenyl-4-hydroxy-naphthpyridin-2(1H)-on-3-carbonsäure-ethylester,

d) 1-Cyclopropylmethyl-4-hydroxy-naphthpyridin-2(1H)-on-3-carbonsäure-ethylester,
e) 1-Allyl-4-hydroxy-naphthpyridin-2(1H)-on-3-carbonsäure-t-butyl-ester mit Smp. 104-107 °C.

Beispiel 4

Eine Lösung von 2.2 g 1-Allyl-4-hydroxy-naphthpyridin-2(1H)-on-3-carbonsäure-t-butylester in 25 ml Acetonitrilwird mit 0.75 ml 60 %-iger Perchlorsäure bei 0 °C behandelt. Der erhaltene Niederschlag wird filtriert und gekühlt und besteht aus 1-Allyl-4-hydroxy-naphthpyridin-2(1H)-on-3-carbonsäure mit Smp. 191-193 °C (Zers.).

**Ansprüche**

1. Verbindung der allgemeinen Formel I,

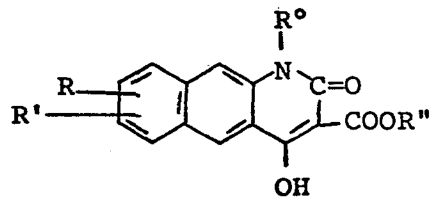

(I)

in der
R° ein Wasserstoffatom, $(C_1—C_6)$-Alkyl, $(C_3—C_6)$-Alkenyl, $(C_3—C_6)$-Alkinyl oder $(C_3—C_6)$-Cycloalkyl, $(C_3—C_6)$-Cycloalkyl, $(C_1—C_2)$-Alkyl oder

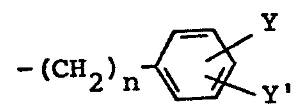

bedeutet, wobei die ungesättigten Stellen in Alkenyl oder Alkinyl sich nicht auf das α-Kohlenstoffatom beziehen,
R" ein Wasserstoffatom oder $(C_1—C_4)$-Alkyl bedeutet,
n 0 oder 1 bedeutet,
Y und Y' unabhängig voneinander ein Wasserstoff-, Fluor-, Chlor- oder Bromatom, $(C_1—C_3)$-Alkyl oder $(C_1—C_3)$-Alkoxy oder Trifluormethyl bedeuten, und
R und R' jeweils ein Wasserstoff-, Fluor-, Chlor- oder Bromatom, $(C_1—C_4)$-Alkyl oder $(C_1—C_4)$-Alkoxy bedeuten,
und deren Mono- und Di-Salze, welche pharmazeutisch akzeptable Kationen enthalten.

2. Verbindung gemäss Anspruch 1, worin R und R' jeweils ein Wasserstoffatom bedeuten.
3. Verbindung gemäss Anspruch 1, worin R° $(C_1—C_6)$-Alkyl bedeutet.
4. Verbindung gemäss Anspruch 1, worin R° $(C_3—C_6)$-Alkenyl bedeutet.
5. Verbindung gemäss Anspruch 1, worin R°

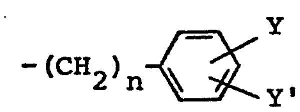

bedeutet.

6. Verbindung gemäss Anspruch 5, worin n = 1 ist.
7. 1-Allyl-4-hydroxy-naphthpyridin-2(1H)-on-3-carbonsäure.
8. Pharmazeutische Zusammensetzung, die einen inerten pharmazeutisch akzeptablen Träger und eine pharmakologisch wirksame Menge einer Verbindung eines der Ansprüche 1 bis 7 enthält.
9. Verfahren zur Herstellung von Verbindungen gemäss einem der Ansprüche 1 bis 7, dadurch gekennzeichnet, dass man eine Verbindung der allgemeinen Formel II,

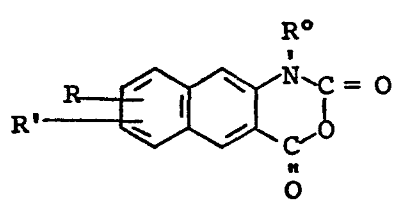

(II)

in der

R, R° und R′ die obenerwähnten Bedeutungen haben, mit einer Verbindung der allgemeinen Formel III

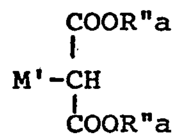

(III)

in der

$R''_a$ $(C_1—C_4)$-Alkyl und

M′ ein Alkalimetallatom bedeutet, umsetzt und gewünschtenfalls zur Bildung einer Verbindung der allgemeinen Formel I, in der R″ ein Wasserstoffatom bedeutet, $R''_a$ durch Reaktion mit einem Säurekatalysator durch ein Wasserstoffatom ersetzt und zur Bildung der Mono- und Di-Salze die so erhaltene Verbindung gewünschtenfalls mit einer Base umsetzt.

10. Verbindungen gemäss einem der Ansprüche 1 bis 7 zur Verwendung als anti-allergische Mittel.

**Claims**

1. Compound of the general formula I

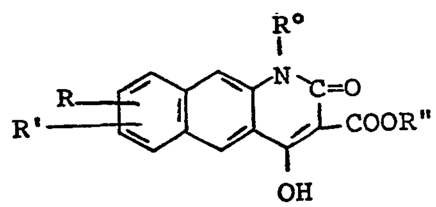

(I)

in which

R° represents a hydrogen atom, $(C_1—C_6)$-alkyl, $(C_3—C_6)$-alkenyl, $(C_3—C_6)$-alkynyl or $(C_3—C_6)$-cycloalkyl, $(C_3—C_6)$-cycloalkyl-$(C_1—C_2)$-alkyl or

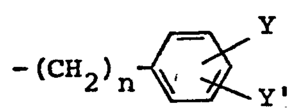

whereby the unsaturated positions in alkenyl or alkynyl are not connected with the α carbon atom,

R″ represents a hydrogen atom or $(C_1—C_4)$-alkyl,

n represents 0 or 1,

Y and Y′ represent independently of each other a hydrogen, fluorine, chlorine or bromine atom, $(C_1—C_3)$-alkyl or $(C_1—C_3)$-alkoxy or trifluoromethyl, and

R and R′ each represent a hydrogen, fluorine, chlorine or bromine atom, $(C_1—C_4)$-alkyl or $(C_1—C_4)$-alkoxy, and their mono- and di-salts which contain pharmaceutically acceptable cations.

2. A compound according to Claim 1, wherein R and R′ each represent a hydrogen atom.

3. Compound according to Claim 1, wherein R° represents $(C_1—C_6)$-alkyl.

4. Compound according to Claim 1, wherein R° represents $(C_3—C_6)$-alkenyl.

5. Compound according to Claim 1, wherein R° represents

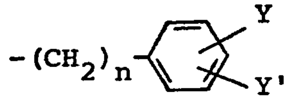

6. Compound according to Claim 5, wherein n is 1.

7. 1-Allyl-4-hydroxy-naphthpyridin-2(1H)-one-3-carboxylic acid.

8. Pharmaceutical composition which contains an inert pharmaceutically acceptable carrier and a pharmacologically active amount of a compound of any one of Claims 1 to 7.

9. Process for the preparation of compounds according to any one of Claims 1 to 7 characterized in that a compound of the general formula II

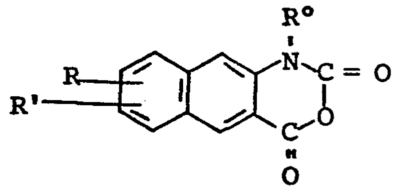

(II)

in which

R, R° and R' have the above mentioned meanings, is reacted with a compound of general formula III

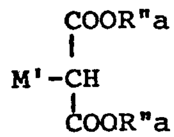

(III)

in which

$R''_a$ represents ($C_1$—$C_4$)-alkyl and

M' represents an alkali metal atom and if desired, to form a compound of the general formula I wherein R" represents a hydrogen atom, $R''_a$ is replaced with a hydrogen atom by reaction with an acid catalyst and, to form the mono- and di-salts, the compound thus obtained is, if desired, reacted with a base.

10. Compounds according to any one of the Claims 1 to 7 for use as anti-allergic agents.

**Revendications**

1. Composé de formule générale I

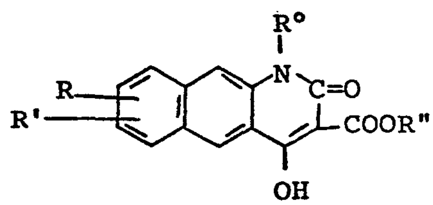

(I)

dans laquelle

R° signifie un atome d'hydrogène, un groupe alkyle contenant de 1 à 6 atomes de carbone, alcényle contenant de 3 à 6 atomes de carbone, alcynyle contenant de 3 à 6 atomes de carbone ou cycloalkyle contenant de 3 à 6 atomes de carbone, cycloalkyl-alkyle dans lequel le reste cycloalkyle contient de 3 à 6 atomes de carbone et le reste alkyle 1 ou 2 atomes de carbone ou

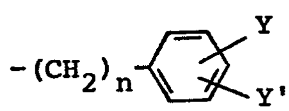

la position insaturée dans le groupe alcényle ou alcynyle ne se rapportant pas à l'atome de carbone α,

R" signifie un atome d'hydrogène ou un groupe alkyle contenant de 1 à 4 atomes de carbone,

n signifie 0 ou 1,

Y et Y' signifient, indépendamment l'un de l'autre, un atome d'hydrogène, de fluor, de chlore ou de brome, un groupe alkyle contenant de 1 à 3 atomes de carbone ou alcoxy contenant de 1 à 3 atomes de carbone ou trifluorométhyle, et

R et R' signifient chacun un atome d'hydrogène, de fluor, de chlore ou de brome, un groupe alkyle contenant de 1 à 4 atomes de carbone ou alcoxy contenant de 1 à 4 atomes de carbone, et leurs mono-sels et di-sels qui contiennent des cations acceptables du point de vue pharmaceutique.

2. Composé selon la revendication 1, dans lequel R et R' signifient chacun un atome d'hydrogène.

3. Composé selon la revendication 1, dans lequel R° signifie un groupe alkyle contenant de 1 à 6 atomes de carbone.

4. Composé selon la revendication 1, dans lequel R° signifie un groupe alcényle contenant de 3 à 6 atomes de carbone.

5. Composé selon la revendication 1, dans lequel R° signifie

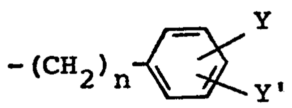

6. Composé selon la revendication 5, dans lequel n signifie 1.

7. L'acide 1-allyl-4-hydroxy-naphtpyridin-2(1H)-one-3-carboxylique.

8. Composition pharmaceutique, qui contient un support inerte acceptable du point de vue pharmaceutique et une quantité pharmacologiquement active d'un composé de l'une des revendications 1 à 7.

9. Procédé de préparation des composés selon l'une des revendications 1 à 7, caractérisé en ce qu'on fait réagir un composé de formule générale II

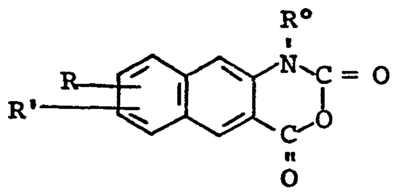

(II)

dans laquelle

R, R° et R' ont les significations mentionnées plus haut, avec un composé de formule générale III

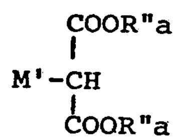

(III)

dans laquelle

$R''_a$ signifie un groupe alkyle contenant de 1 à 4 atomes de carbone et

M' un atome de métal alcalin, et si on désire obtenir un composé de formule générale I dans laquelle R'' signifie un atome d'hydrogène, on remplace $R''_a$ par un atome d'hydrogène par réaction avec un catalyseur acide, et pour obtenir les mono- et di-sels on fait réagir si on le désire le composé ainsi obtenu avec une base.

10. Composés selon l'une des revendications 1 à 7 pour l'utilisation comme agents anti-allergiques.